(19) Europäisches Patentamt
European Patent Office
Office européen des brevets

(11) **EP 1 605 881 B1**

(12) **EUROPEAN PATENT SPECIFICATION**

(45) Date of publication and mention
of the grant of the patent:
**10.04.2013 Bulletin 2013/15**

(51) Int Cl.:
*A61L 15/42* *(2006.01)*      *A61F 13/535* *(2006.01)*
*A61L 15/60* *(2006.01)*

(21) Application number: **04702888.1**

(22) Date of filing: **16.01.2004**

(86) International application number:
**PCT/SE2004/000049**

(87) International publication number:
**WO 2004/084785 (07.10.2004 Gazette 2004/41)**

(54) **ABSORBENT ARTICLE COMPRISING AN ABSORBENT STRUCTURE**

SAUGFÄHIGER GEGENSTAND MIT ABSORPTIONSSTRUKTUR

ARTICLE ABSORBANT COMPRENANT UNE STRUCTURE ABSORBANTE

(84) Designated Contracting States:
**AT BE BG CH CY CZ DE DK EE ES FI FR GB GR HU IE IT LI LU MC NL PT RO SE SI SK TR**

(30) Priority: **26.03.2003 SE 0300879**

(43) Date of publication of application:
**21.12.2005 Bulletin 2005/51**

(73) Proprietor: **SCA Hygiene Products AB**
**405 03 Göteborg (SE)**

(72) Inventors:
• **GUIDOTTI, Ted**
**S-412 67 GÖTEBORG (SE)**
• **MAGNUSSON, Ing-Britt**
**S-435 37 MÖLNLYCKE (SE)**
• **PEHRSON, Madeleine**
**S-435 42 MÖLNLYCKE (SE)**

(74) Representative: **Egeröd, Lisbeth**
**Valea AB**
**Lindholmspiren 5**
**417 56 Gothenburg (SE)**

(56) References cited:
WO-A1-01/15649        WO-A1-99/55393
WO-A1-2004/007598     US-A- 5 338 766
US-A- 5 817 704

## Description

### Field of the invention

The present invention relates to an absorbent article such as a diaper, an incontinence guard, a sanitary napkin or the like, whereby the article exhibits a liquid permeable upper surface and comprises an absorbent structure, exhibiting a planar extension, wherein the absorbent structure comprises an acquisition layer and at least one storage layer.

### Background

Absorbent articles such as diapers, incontinence guards, sanitary napkins, intended for one single use, usually comprise an absorbent structure having the capability to acquire large amounts of liquid under a short period of time, having further the ability to distribute the liquid and to store the liquid. This means that the absorbent structure usually comprises several different layers having different properties with respect to each other. Frequently, the absorbent structure at least comprises a liquid acquisition layer and a liquid storage layer. The liquid storage layer often comprises a cellulosic fluff pulp layer mixed with a super absorbent material, which are polymers having the ability to absorb water or bodily fluids many times their own weight. The liquid acquisition layer often comprises a porous fibrous layer of synthetic fibres.

Upon usage of such absorbent articles, it is desirable that they are thin and discrete to wear, and that they at the same time rapidly can acquire a large amount of liquid discharged during a short period of time and then store this liquid in the article.

However, it has been shown that it is difficult to obtain sufficient liquid acquiring capacity, liquid distribution capacity and liquid storage capacity for products, which at the same time are thin and discrete to wear.

In order to obtain a thin product, it is previously known to use a relatively high percentage of a super absorbent material in the absorbent structure. By for example EP 0 532 002 and EP 0 615 736, it is known to use an absorbent structure for use in a diaper containing at least 30 percent by weight or more of a super absorbent material. It is further known from EP 0 443 627 to use an absorbent structure containing 60 percent by weight of a super absorbent material.

However, it has been shown to be difficult to obtain a thin absorbent article exhibiting an optimal combination of sufficiently high liquid acquiring capacity, sufficient local and total absorption capacity and sufficient liquid transport ability. It is further important, mainly for diapers and incontinence guards, that the article upon repeated wettings is able to receive and absorb relatively large amounts of liquid discharged during a short period of time.

### Description of the invention

According to the present invention, an article has been provided, which is thin and discrete to wear, and at the same time the article should exhibit sufficient liquid acquiring capacity, liquid distribution capacity and liquid storage capacity.

An absorbent article according to the invention is characterised mainly in that the acquisition layer comprises a plurality of fragments of a liquid absorbing open-celled polyacrylate-based foam material, wherein each fragment exhibits a planar extension having a longitudinal direction and a transversal direction, and a thickness direction extending perpendicularly to the planar extension, wherein the width in the transversal direction on each fragment in a dry condition, does not exceed 10 millimetres and that the total area of the fragments in the planar extension is less than the total area of the absorbent structure in the planar extension. Since the acquisition layer comprises a plurality of separate fragments, where at least most fragments are arranged with a distance to each other, a free space between the fragments is obtained. This results in that the fragments during expansion can expand unhindered. Due to the free spaces between the fragments, the foam fragments have thereby the ability to expand in three dimensions upon wetting, i.e., in all three planes of the fragments.

According to one embodiment, the width of the fragments is not more than 7.0 millimetres and more preferred, not more than 6.0 millimetres.

According to one embodiment, each fragment exhibits in a dry condition a length, i.e., in the longitudinal direction of the fragments, which does not exceed 20 millimetres or more preferred, not more than 10 millimetres.

Upon storage of absorbent articles in a sealed diaper package, the thickness of the fragments is preferably below 2 millimetres. The term "diaper package" denotes the package, in which the diapers are enclosed when selling the diapers. In some cases, the diapers are packed one by one, whereby a number of single-wrapped diapers then are further enclosed in a bigger package. Thus, the term "diaper package" does not denote the single-wrapped diaper, but the bigger diaper package. Analogously, when the absorbent article is an incontinence guard or a sanitary napkin, the thickness of the fragments is referred to as the exhibited thickness in the absorbent structure comprised in said articles within a sealed package for incontinence guards or sanitary napkins. Upon measurement of the thickness of the fragments, the measurement must be performed within 2 minutes after opening the package in question.

Preferably, the fragments are obtained in such a way that a plurality of parts, i.e., fragments, are cut out from a compressed foam layer. Thus, the thickness of the fragments in a dry condition is equal to the thickness of the compressed foam layer In a dry condition. The polyacrylate-based foam material is highly compressed in a dry condition, which leads to that it expands heavily upon wetting. The fragments are cut out in such a way so that they substantially exhibit a rectangular shape.

According to an alternative way to produce the fragments, the foam is dripped into separate drops, whereby the foam structure is then being cross-linked, compressed and dried. Upon such production of the fragments, the fragments obtain a circular or oval shape.

According to one embodiment, the total area of the fragments in the planar extension is maximally 50 % of the total area of the absorbent structure in the planar extension. According to yet another embodiment, the total area of the fragments in the planar extension is maximally 30 % of the total area of the absorbent structure in the planar extension

It is also possible that some portions of the absorbent structure exhibit a plurality of fragments, whilst other portions of the absorbent structure are essentially free from fragments. For example, it is possible that the fragments are arranged against the upper surface of the absorbent structure in the crotch portion, whilst the two end portions at least substantially are free from fragments. However, according to that embodiment, single fragments may appear outside the portion intended to contain fragments. In an embodiment having one or more portions with fragments, it is the total area of the fragments in the planar extension which preferably maximally is 50 % of the total area in the planar extension. The size of a portion having fragments is estimated in the following way. The closest distance to an adjacent fragment should not be larger than five times the width of the fragment. If the closest distance to an adjacent fragments is more than five times the width of this fragment, thus the fragment in question does not belong to the part being the area of the portion.

According to a further embodiment, the total area of the fragments in a portion is maximally 30 % of the total area of the absorbent structure in such portion.

According to another embodiment, the free space between several fragments is at least twice as large as the width of the fragments. The free space between the fragments, i.e., the distance between fragments, is measured between the outer edges of the fragments. The term "several fragments" denotes at least 70 % of the total number of fragments and more preferred at least 80 % of the total number of fragments.

It has shown to be especially advantageous to design the absorbent structure in such a way that the foam fragments are located in the portion being closest to the user, i.e., in the portion being the first one to be wet. The advantage using such an acquisition layer, is that it is able to receive a large amount of liquid in a short period of time. The cell walls in the foam fragments expands heavily upon wetting. When the cell walls of the foam material expand, the volume in the pores heavily increases. The liquid absorbed by the pores is very loosely bound and can be drained by a underlying layer having higher capillary forces. Consequently, the acquisition layer can receive a second dose of liquid, i.e., a new dose of liquid discharged during a short period of time.

The part of the liquid absorbed by the cell walls is more tightly bound than the loosely bound liquid absorbed by the pores. It has been proved to be an advantage that a certain amount of the liquid is absorbed by the cell walls and thereby is tightly bound. The risk is then smaller than for other acquisition layers that the surface closest to the user is wetted.

Upon storage of absorbent articles in a sealed diaper package, the thickness of the fragments is preferably exceeding 0.50 $g/cm^3$. The term "diaper package" denotes the package, in which the diapers are enclosed when selling the diapers. In some cases, the diapers are packed one by one, whereby a number of single-packed diapers then are further enclosed in a bigger package. The term "diaper package" does not denote a singly-packed diaper but the bigger diaper package. Analogously, when the absorbent article is an incontinence guard or a sanitary napkin, the density of the fragments is referred to as the exhibited density in the absorbent structure comprised in said articles within a sealed package for incontinence guards or sanitary napkins. Measurements of the density of the fragments must be made within 2 minutes after opening the package in question.

According to one embodiment, the volume of each fragment at least increases with 500 % upon wetting.

According to another embodiment, the area in the planar extension of each fragment upon wetting increases with at least 300 %.

The polyacrylate-based super absorbent foam material is produced by the saturation under pressure using carbon dioxide of a solution, which at least contains monomer, a cross-linking material, an initiator and a tenside in a vessel. When the solution is removed from the vessel through a nozzle, the solution is expanded and a foamed structure is achieved. The foamed structure is then locked in that polymerisation and cross-linking are initiated by for instance UV radiation and/or e-beam. Finally, the material is compressed and dried.

It has been shown that the plurality of fragments can be applied and be maintained against a surface without the need of applying any kind of adherent material.

It is also possible to apply the fragments of the super absorbent foam material, i.e., the acquisition layer, between two storage layer. The fragments can be applied equally distributed of the entire surface of the storage layer, solely in the crotch portion or intermittent in some portions, such as for example in longitudinally or transversally extending strands.

According to an embodiment of an absorbent article, the storage layer comprises cellulosic fibres and a particulate super absorbent material, wherein the amount particulate super absorbent material, calculated on the total weight of the storage layer in a dry condition is at least 50 percent by weight. The percentage super absorbent material calculated on the total weight of the storage layer in a dry condition can be higher, such as at least 60 percent by weight or at least 70 percent by weight. In order to create an thin article, which is discrete and comfortable to wear and which at the same time exhibits a sufficiently high absorption capacity, it has been shown that it is advantageous to use a high percentage of the super absorbent material in the storage layer.

**Brief description of the drawings**

Fig. 1 shows a planar view of an absorbent article according to the invention.

Fig. 2 shows a cross-section of the absorbent article shown in Fig. 1.

Fig. 3 shows a cross-section of an alternative embodiment of an absorbent article according to the invention.

**Detailed description of the drawings**

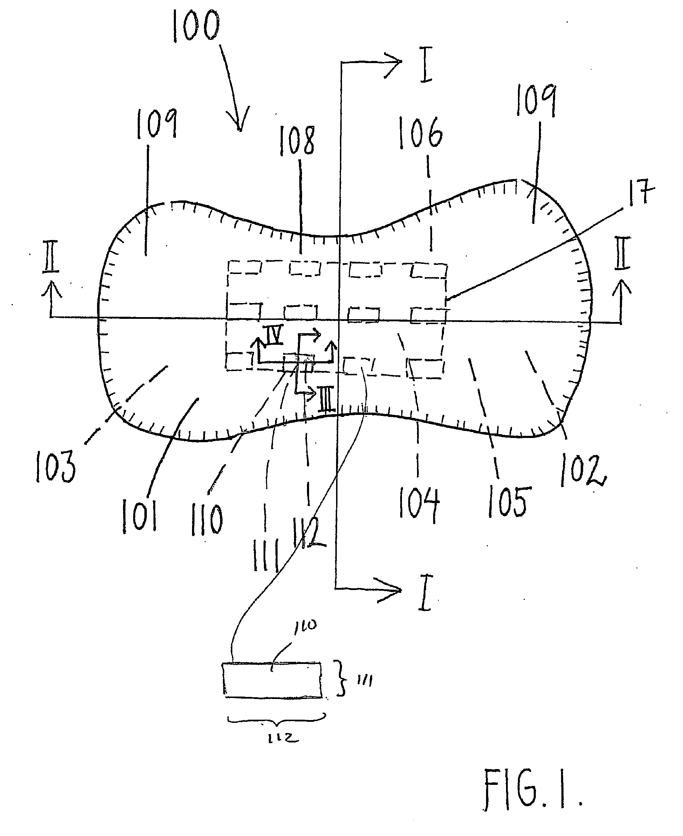

The following description refers to a couple of embodiments of absorbent articles according to the invention, which is thus not limited to the below described embodiments. In Figure 1 a planar view of an absorbent article 100 according to the invention is shown. The absorbent article 100 exhibits a transversal direction, being shown by a transversally extending centre line I, and a longitudinal direction being shown by a longitudinally extending centre line II. Further, the absorbent article 100 exhibits a thickness direction, being perpendicular to the plane. The absorbent article 100 has a liquid permeable top sheet 101, which during use of the article is intended to lie closest to the user. Further, the absorbent article 100 has a back sheet 102, which is at least substantially liquid impermeable, and an absorbent structure 103 enclosed between the liquid permeable top sheet and the back sheet. The back sheet material 102 may optionally be a so called vapour permeable breathable material. The absorbent structure 103 comprises an acquisition layer 104, which is intended to rapidly be able receive a large amount of liquid and a first storage layer 105, which is intended to rapidly be able to store a large amount of liquid, and a second storage layer 106. The second storage layer 106 has a longer extension in the plane of the article than the first storage layer 105, but exhibits a lower total absorption capacity. The second storage layer also functions as a form rendering element in such way that it assists in creating and maintaining an absorbent structure being flexible against the body.

The storage layers 105, 106 may comprise optional absorbent materials, such as fibrous materials, foam materials, superabsorbent polymers and combinations of these. According to one embodiment the first storage layer 105 is a fibrous structure comprising a relatively high proportion, preferably at least 50 percent by weight of a super absorbent material calculated on the total weight of the first storage layer 105. Super absorbent material are polymers having the capability to absorb water or bodily fluids many times their own weight. The super absorbent material is present in the form of powder, flakes, fibres, granules or the like. The super absorbent material in the storage layer 105 may be mixed with the fibre material or may be applied as one or more layers between fibre layers. The super absorbent material is either equally distributed in the first storage layer 105 or distributed in various concentrations in the longitudinal and/or the thickness direction of the first storage layer 105.

The second storage layer 106 is preferably thinner than the first storage layer 105. Also the second storage layer 106 can according to an embodiment comprise a fibrous structure containing superabsorbent material. The percentage super absorbent material in the second storage layer 106 is preferably lower than the amount of super absorbent material in the first storage layer 105. For example, the second storage layer 106 may comprise about 10 percent by weight of a super absorbent material calculated on the total weight of the second storage layer 106. The second storage layer 106 has a longer extension in the plane of the article, but exhibits a lower total absorption capacity. The liquid permeable top sheet 101 can be a nonwoven material or an apertured plastic film, or a laminate thereof. Examples of polymers of which the liquid permeable top sheet can be made of is polyethylene, polypropylene, polyester, or copolymers thereof. To enable the liquid permeable top sheet 101 to rapidly let the discharged bodily fluid through, the top sheet is often coated with tensides and/or is apertured.

The acquisition layer 104 comprise a plurality of fragments 110 made of an open-celled polyacrylate-based foam material, wherein each fragment 110 exhibits a planar extension, exhibiting a longitudinal direction being shown by a longitudinally extending centre line IV, and a transversal direction being shown by a transversally extending centre line III. Further, each fragment 110 exhibits a thickness direction being perpendicular to the planar extension. The width 111 in the transversal direction on each fragment 110 in a dry condition, does not exceed 10 millimetres. The fragments 110 of the acquisition layer 104 further exhibit a length 112, which does not exceed 20 millimetres. The fragments 110 in the acquisition layer 104 are arranged in a portion of the crotch portion 108 of the absorbent structure. The outermost fragments in the portion containing the fragments determines the total area of the portion, In order to determine the area of that portion of the absorbent structure, a line is drawn between the outer edges of outermost fragments. This portion is indicated with a dotted line 17 in Figure 1.

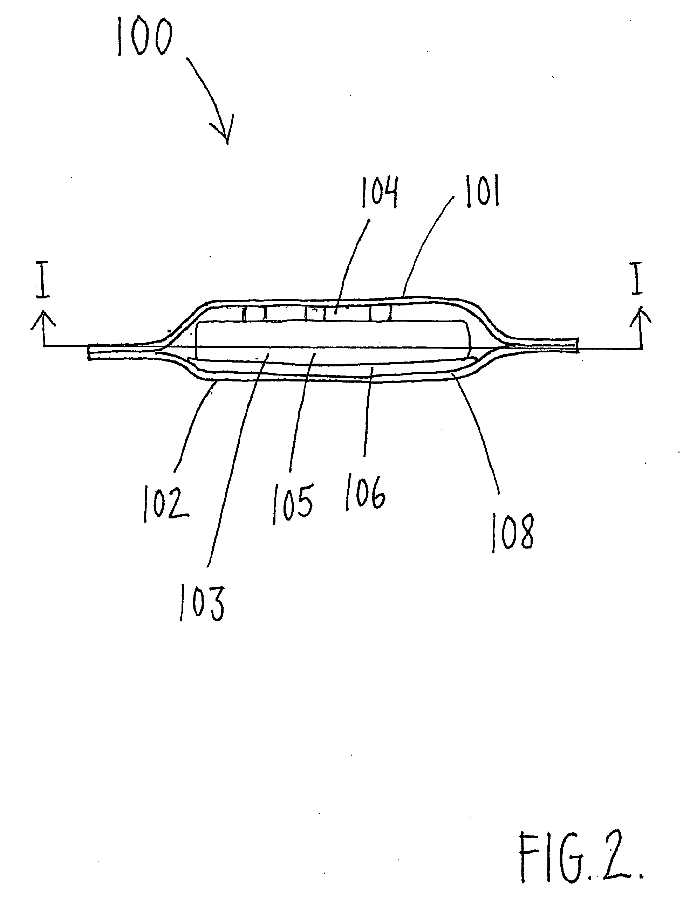

Figure 2 shows a cross-section in the crotch portion 108 of the absorbent article 100 shown in Figure 1. Thus, the absorbent article 100 has a liquid permeable top sheet 101, which during use of the article is intended to lie closest to the user, a substantially liquid impermeable back sheet 102, and an absorbent structure 103 enclosed therebetween. The absorbent structure 103 comprises an acquisition layer 104, which is arranged closest towards the liquid permeable top sheet 101, a first storage layer 105, and a second storage layer 106. The first storage layer 105, is arranged between the acquisition layer 104 and the second storage layer 106. The second storage layer 106 is arranged between the first storage layer 105 and the backsheet 102. The construction of the different layers is described in detail in the description of Figure 1.

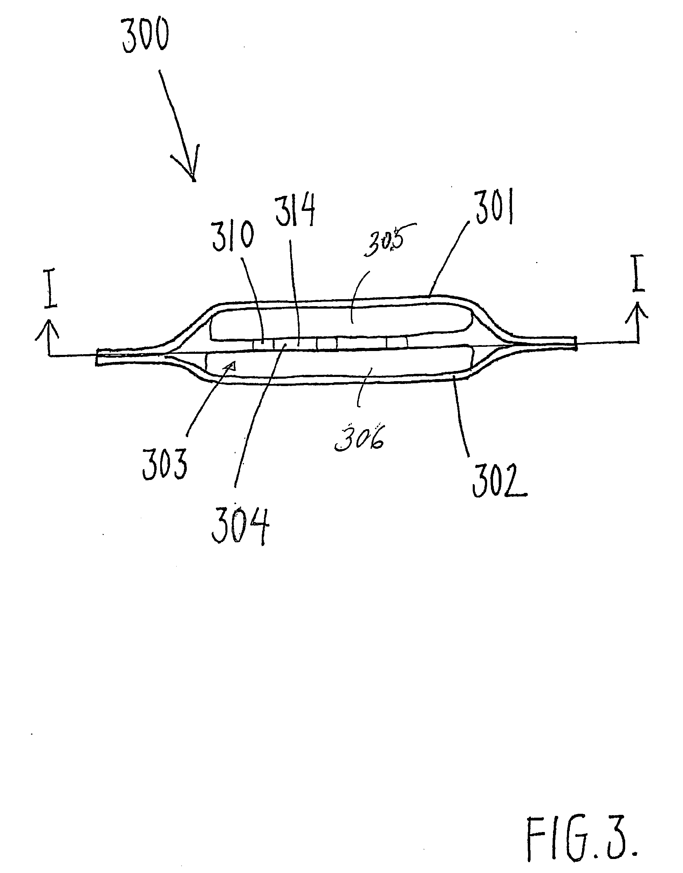

Fig. 3 shows a cross-section along a transversally extending centre line I of an alternative embodiment of an absorbent article 300 according to the invention. The absorbent article 300 has a liquid permeable top sheet 301, which during use of the article 300 is intended to lie closest to the user, a substantially liquid impermeable back sheet 302, and an absorbent structure 303, 304, 305 enclosed therebetween. An acquisition layer 304 comprises three separate fragments 310. The fragments is constituted of strips extending in the longitudinal direction of the article. Between the strips, there is a space being free from the acquisition material, i.e., a hollow space, 314. Preferably the strips comprise a super absorbent foam material. Upon wetting such a super absorbent expands heavily in all directions of the material. Thus, the hollow spaces 314 render space for the super absorbent foam material to expand without substantially change the shape of the absorbent structure.

The storage layer is divided in an upper and a lower storage layer 305 and 303, at which the acquisition layer 304 is placed between these two layers. The upper and the lower storage layers may be of the same material compositions as has been stated above with respect to the storage layers 105 and 106 in Fig. 1. The material composition can be the same in the two layers 303 and 305, or it can be different, for example may the content of cellulosic fluff pulp and superabsorbent material be different in the different layers.

**Example 1 - Measurements of the expansion of foam materials**

Upon measurement of the expansion in the thickness direction of the materials, samples were cut out having a known diameter. The dry diameter ($d_d$) and the dry thickness ($t_d$) were measured. The samples were then allowed to swell in a NaCl solution (0.9 grams by weight of NaCl). Thereafter, the wet diameter ($d_w$) and the wet thickness ($t_w$) were measured. The expansion of the thickness (TE) expressed in percentage, the expansion of the area (AE) expressed in percentage and the expansion of the volume (VE) expressed in percentage, were then estimated using the formulas below.

$$TE = (t_w - t_d)/t_d * 100$$

$$AE = (d_w^2 - d_d^2)/d_d^2 * 100$$

$$VE = (t_w d_w^2 - t_d d_d^2)/t_d d_d^2 * 100$$

The tested materials are a polyacrylate-based foam material, denoted Foam XII, Foam XII has been made according to the following description:

To a beaker the following is added:
348.5 grams of acrylic acid (4.84 moles)
135.5 grams of a sodium acrylate solution containing 37.3 percent per weight (0.54 moles)
28.0 grams of polyethylene glycol diacrylate from polyethylene glycol having a molecular weight of 400.
21.3 grams of an aqueous solution 15 percent per weight containing ethylene oxide and linear $C_{16}$-$C_{18}$ fatty alcohol (molar ratio 80: 1)
65.7 grams of water.

The ingredients were mixed and thereafter, the solution was cooled to a temperature lower than 16 °C. The solution was then poured into a closed container, whereby the solution was saturated with carbon dioxide at a pressure of 12 bar for 25 minutes. Using the same pressure, 26.7 grams of an aqueous solution containing 3 percent by weight of 2,2'-azobis(2-amidinopropane) dihydrochloride. This was mixed to a homogenous solution. The solution was then allowed to rest in five minutes. The saturated solution was compressed from a container using a nozzle having an opening being 1 mm at a pressure being 12 bar.

The resulting monomeric foam was placed on a glass plate (DIN-A3). An additional glass plate was then placed on top of the monomeric foam. Then, the foam was polymerised using a UV/VIS lamp, a UV1000 lamp from Höhnle. The foam was illuminated using the lamp both from underneath and from above. The illumination and thereby also the polymerisation was allowed to proceed for 4 minutes.

**Table 1**

| $d_d$ | $t_d$ (s) | $d_w$ | $t_w$ (s) | TE | AE | VE |
|---|---|---|---|---|---|---|
| 28 | 3.8 | 62 | 6 | 58 | 357 | 622 |
| 28 | 3.8 | 61 | 6 | 58 | 390 | 577 |
| 28 | 3.8 | 62 | 6 | 58 | 390 | 649 |

The thickness expansion should be preferably 40 -80 %, the area expansion should preferably be 300-400 % and the volume expansion should preferably be 500-700 %.

**Example 2 - Estimation of the time required for an absorbent article to absorb a predetermined amount of liquid**

Measurements have been performed to determine the time required for five different absorbent articles to absorb 100 ml of synthetic urine. Further, the time required for an additional second and third liquid dose to be absorbed was also measured, which corresponds to a second and third wetting, respectively.

All five absorbent articles comprise a top sheet, acquisition layer, storage layer and backsheet. Because the measurement related to the time required for the absorbent article to absorb a predetermined amount of liquid, the tested articles did not exhibit any elastic means or fastening means. Thus, it is the function of the topsheet and the absorbent structure which is being tested. The top sheet in all five tested articles is made of a carded nonwoven material of polypropylene denoted "Novelin 650", from JW Suominen OY. The grammage is 23 g/m$^2$. The back sheet consists in all five tested articles of a polypropylene film.

Tested absorbent article No. 1

The absorbent structure comprises a acquisition layer containing a plurality of fragments of Foam XII. The fragment have a width being 5 mm and a length of 7 mm. The total weight of the fragments, i.e., the weight of the acquisition layer is 2.08 grams, which during manufacture may vary 0.01 grams. The acquisition layer is arranged between the liquid permeable top sheet and the storage layer. The storage layer comprises a first storage layer and a second storage layer. Both the first storage layer and the second storage layer consists of cellulosic fluff pulp and a particulate polyacrylate-based super absorbent material. The cellulosic fluff pulp in the first storage layer is a chemically manufactured pulp from Weyerhauser and is denoted NB 416. The cellulosic fluff pulp is formed in three different layers, wherein the layers have a grammage being 100 g/m$^2$, 50 g/m$^2$ and 100 g/m$^2$, respectively. The particulate super absorbent material is a polyacrylate-based super absorbent material. The particulate super absorbent material is applied between the cellulosic layers, whereby the super absorbent then consists of two separate layers. Each layer of the particulate super absorbent material exhibits a grammage being 275 g/m$^2$. The second storage layer comprises a chemically manufactured pulp and polyacrylate-based super absorbent material. The super absorbent is mixed in the cellulosic fluff pulp. The percentage of super absorbent material in the second storage layer is 10 percent by weight. The total grammage of the second storage layer is 200 g/m$^2$.

Tested absorbent article No. 2

The absorbent structure comprises a acquisition layer containing a layer of Foam XII. The acquisition layer has a width being 6 cm and a length of 13 cm, and the total weight of the acquisition layer is 6.25 grams. The acquisition layer is arranged between the liquid permeable top sheet and the storage layer. The storage layer comprises the same structure as the storage layer of the tested absorbent article No. 1.

Tested absorbent article No. 3

Acquisition layer containing a plurality of fragments of Foam XII. The acquisition layer is arranged between two different layers of storage material. The storage layer comprises the same structure as the storage layer of the tested absorbent article No. 1.

Tested absorbent article No. 4

Acquisition layer containing a layer of Foam XII, wherein the acquisition layer is arranged between two different layers of storage material. The storage layer comprises the same structure as the storage layer of the tested absorbent article No. 1.

Tested absorbent article No. 3

Finally, an article was tested that did not contain any acquisition layer. The absorbent article consisted of a storage layer, which was constructed in the same way as the storage layer of the tested absorbent article No. 1.
The time required to absorb 100 ml of synthetic urine was measured. Further, the time required for an additional second and third liquid dose to be absorbed was also measured, which corresponds to a second and third wetting, respectively.

**Table 2**
**Absorption time (s)**

| Wetting | Art.1 | Art. 2 | Art. 3 | Art. 4 | Art. 5 |
|---------|-------|--------|--------|--------|--------|
| No. 1 | 7.4 | 22.8 | 15.3 | 17.2 | 22.5 |
| No. 2 | 18.3 | 60.9 | 38.8 | 43.2 | 33.4 |
| No. 3 | 29.6 | 81.6 | 62.8 | 67.1 | 56.1 |

The results show that Article 1 exhibited the shortest absorption time at all three wettings. Article 1 comprised a acquisition layer made of a plurality of fragments of Foam XII. The acquisition layer in Article 1 was arranged between a liquid permeable top sheet and a storage layer.

**Example 3 - Estimation of the rewetting**

The rewetting, i.e., how wet a top sheet of an absorbent article is after wetting, has been measured for three absorbent articles.
The top sheet of all three tested articles was constituted of a carded nonwoven material of polypropylene having a grammage of 23 $g/cm^2$. The manufacturer of the top sheet is JW Suominen OY. The storage material in all three tested articles was constituted of five layers of a reference filter paper denoted ERT FF3 from Hollingsworth Vose Company Ltd. Test liquid for all test samples was a solution containing 9 percent by weight of NaCl.
The first tested absorbent article comprised an acquisition layer constituted of a plurality of fragments of Foam XII. The width of the fragments was 5 mm ands the length of the fragments was 5 mm. The total weight of the fragments was 1.0 grams.
The second tested absorbent article comprised an acquisition layer constituted of a plurality of fragments of a compressed foam material from regenerated cellulose from Polyform GmbH. The width of the fragments was 7 mm ands the length of the fragments was 7 mm. The total weight of the fragments was 1.0 grams.
The third tested absorbent article did not comprise any acquisition layer at all.

**Table 3**

| Article | Rewetting (grams) |
|---------|-------------------|
| No. 1 | 0.10 |
| No. 2 | 1.46 |
| No. 3 | 0.13 |

The results show that Article 1 provided the lowest rewetting. Article 1 comprised an acquisition layer being constituted of fragments of the polyacrylate-based foam material Foam XII.

**EP 1 605 881 B1**

**Claims**

1. Absorbent article (100) such as a diaper, an incontinence guard, a sanitary napkin or the like, whereby the article (100) exhibits a liquid permeable upper surface and comprises an absorbent structure (103), exhibiting a planar extension, wherein the absorbent structure (103) includes an acquisition layer (104) and at least one storage layer (106), *characterised in,* **that** the acquisition layer (104) comprises a plurality of fragments (110) of a liquid absorbing open-celled polyacrylate-based foam material, wherein each fragment (110) exhibits a planar extension having a transversal direction (III) and a longitudinal direction (IV), and a thickness direction extending perpendicularly to the planar extension, wherein the width in the transversal direction (III) on each fragment (110) in a dry condition does not exceed 10 millimetres, and that the total area of the fragments in dry condition in the planar extension is lower than the area of the absorbent structure in the planar extension.

2. Absorbent article according to claim 1, *characterised in,* **that** each fragment (110) in a dry condition exhibits a width in the transversal direction (III), which does not exceed 7 millimetres.

3. Absorbent article according to claim 1 or claim 2, *characterised in,* **that** each fragment (110) in a dry condition exhibits a length in the longitudinal direction (IV), which does not exceed 20 millimetres.

4. Absorbent article according to any of the claims 1-3, *characterised in,* **that** the total area of the fragments (110) in the planar extension maximally is 50 % of the total area of the absorbent structure (103) in the planar extension.

5. Absorbent article according to any of the claims 1-3, *characterised in,* **that** the total area of the fragments (110) in the planar extension maximally is 30 % of the total area of the absorbent structure (103) in the planar extension.

6. Absorbent article according to any of the preceding claims, *characterised in,* **that** each fragment (110) in a dry condition exhibits a density being at least 0.18 g/cm$^3$.

7. Absorbent article according to any of the preceding claims, *characterised in,* **that** the volume of each fragment (110) upon wetting at least increases with 500 %.

8. Absorbent article according to any of the preceding claims, *characterised in,* **that** the area in the planar extension of each fragment (110) upon wetting at least increases with 300 %.

9. Absorbent article according to any of the preceding claims, *characterised in,* **that** the fragments (110) are applied against the upper surface of the storage layer (106), at least in the wetting area.

10. Absorbent article according to any of the preceding claims, *characterised in,* **that** at least one of the storage layers (106) is comprised of cellulosic fibres and particulate super absorbent, wherein the amount super absorbent material calculated on the total weight of the storage layer (106) in dry condition is at least 50 percent by weight.

11. Absorbent article according to any of the claims 1-9, *characterised in,* **that** at least one of the storage layers (106) is comprised of cellulosic fibres and particulate super absorbent, wherein the amount super absorbent material calculated on the total weight of the storage layer (106) in dry condition is at least 70 percent by weight.

**Patentansprüche**

1. Saugfähiger Artikel (100), wie zum Beispiel eine Windel, ein Inkontinenzschutz, eine Damenbinde oder Ähnliches, wobei der Artikel (100) eine flüssigkeitsdurchlässige obere Fläche und eine eine plane Erstreckung aufweisende saugfähige Struktur (103) aufweist und die saugfähige Struktur (103) eine Aufnahmelage (104) und mindestens eine Speicherlage (106) aufweist, **dadurch gekennzeichnet, dass** die Aufnahmelage (104) eine Vielzahl von Fragmenten (110) eines flüssigkeitsabsorbierenden offenzelligen polyacrylatbasierten Schaummaterials aufweist, wobei jedes Fragment (110) eine plane Erstreckung mit einer Querrichtung (III) und einer Längsrichtung (IV) und einer sich senkrecht zu der planen Erstreckung erstreckende Dickenrichtung aufweist, wobei die Breite in der Querrichtung (III) auf jedem Fragment (110) in einem trockenen Zustand 10 Millimeter nicht überschreitet und die Gesamtfläche der Fragmente in trockenem Zustand in der planen Erstreckung kleiner ist als die Fläche der saugfähigen Struktur in der planen Erstreckung.

**2.** Saugfähiger Artikel nach Anspruch 1, **dadurch gekennzeichnet, dass** jedes Fragment (110) in trockenem Zustand eine Breite in der Querrichtung (III) aufweist, die 7 mm nicht überschreitet.

**3.** Saugfähiger Artikel nach Anspruch 1 oder 2, **dadurch gekennzeichnet, dass** jedes Fragment (110) in trockenem Zustand eine Länge in der Längsrichtung (IV) aufweist, die 20 mm nicht überschreitet.

**4.** Saugfähiger Artikel nach einem der Ansprüche 1 bis 3, **dadurch gekennzeichnet, dass** die Gesamtfläche der Fragmente (110) in der planen Erstreckung maximal 50 % der Gesamtfläche der saugfähigen Struktur (103) in der planen Erstreckung beträgt.

**5.** Saugfähiger Artikel nach einem der Ansprüche 1 bis 3, **dadurch gekennzeichnet, dass** die Gesamtfläche der Fragmente (110) in der planen Erstreckung maximal 30 % der Gesamtfläche der saugfähigen Struktur (103) in der planen Erstreckung beträgt.

**6.** Saugfähiger Artikel nach einem der vorstehenden Ansprüche, **dadurch gekennzeichnet, dass** jedes Fragment (110) in einem trockenen Zustand eine Dichte von mindestens 0,18 g/cm$^3$ aufweist.

**7.** Saugfähiger Artikel nach einem der vorstehenden Ansprüche, **dadurch gekennzeichnet, dass** das Volumen von jedem Fragment (110) bei Befeuchtung um mindestens 500 % ansteigt.

**8.** Saugfähiger Artikel nach einem der vorstehenden Ansprüche, **dadurch gekennzeichnet, dass** die Fläche der planen Erstreckung von jedem Fragment (110) bei Befeuchtung um mindestens 300 % ansteigt.

**9.** Saugfähiger Artikel nach einem der vorstehenden Ansprüche, **dadurch gekennzeichnet, dass** die Fragmente (110) zumindest in dem Befeuchtungsbereich gegen die obere Fläche der Speicherlage (106) aufgebracht werden.

**10.** Saugfähiger Artikel nach einem der vorstehenden Ansprüche, **dadurch gekennzeichnet, dass** mindestens eine der Speicherlagen (106) aus Zellulosefasern und partikelförmigen Superabsorbern ausgebildet ist, wobei die Menge an superabsorbierendem Material, die für das gesamte Gewicht der Speicherlage (106) in trockenem Zustand berechnet wird, mindestens 50 Gewichtsprozent beträgt.

**11.** Saugfähiger Artikel nach einem der Ansprüche 1 bis 9, **dadurch gekennzeichnet, dass** mindestens eine der Speicherlagen (106) aus Zellulosefasern und partikelförmigen Superabsorbern zusammengesetzt ist, wobei die Menge an superabsorbierendem Material, die auf Grundlage des Gesamtgewichts der Speicherlage (106) in trockenem Zustand berechnet wird, mindestens 70 Gewichtsprozent beträgt.

**Revendications**

**1.** Article absorbant (100) tel qu'une couche-culotte, une protection contre l'incontinence, une serviette hygiénique ou un article similaire, l'article (100) présentant une surface supérieure perméable aux liquides et comprenant une structure absorbante (103) qui présente une étendue plane, la structure absorbante (103) renfermant une couche de réception (104) et au moins une couche de stockage (106), *caractérisé en ce que* la couche de réception (104) comprend une pluralité de fragments (110) d'un matériau du type mousse à base de polyacrylate à alvéoles ouvertes qui absorbe les liquides, chaque fragment (110) présentant une étendue plane qui comporte une direction transversale (III) et une direction longitudinale (IV) ainsi qu'une direction de l'épaisseur qui s'étend perpendiculairement à l'étendue plane, la largeur dans la direction transversale (III) sur chaque fragment (110) à l'état sec ne dépassant pas 10 millimètres et la surface totale des fragments à l'état sec dans l'étendue plane étant inférieure à la surface de la structure absorbante dans l'étendue plane.

**2.** Article absorbant selon la revendication 1 *caractérisé en ce que* chaque fragment (110) présente à l'état sec une largeur, dans la direction transversale (III), qui ne dépasse pas 7 millimètres.

**3.** Article absorbant selon la revendication 1 ou 2 *caractérisé en ce que* chaque fragment (110) présente à l'état sec une longueur, dans la direction longitudinale (IV) qui ne dépasse pas 20 millimètres.

**4.** Article absorbant selon l'une quelconque des revendications 1 à 3 *caractérisé en ce que* la surface totale des fragments (110) dans l'étendue plane, est au maximum de 50 % de la surface totale de la structure absorbante (103) dans l'étendue plane.

5. Article absorbant selon l'une quelconque des revendications 1 à 3 *caractérisé en ce que* la surface totale des fragments (110) dans l'étendue plane est au maximum de 30 % de la surface totale de la structure absorbante (103) dans l'étendue plane.

6. Article absorbant selon l'une quelconque des revendications qui précèdent *caractérisé en ce que* chaque fragment (110) présente, à l'état sec, une masse volumique d'au moins 0,18 g/cm$^3$ .

7. Article absorbant selon l'une quelconque des revendications qui précèdent *caractérisé en ce que* le volume de chaque fragment (110) augmente au moins de 500 % lors du mouillage.

8. Article absorbant selon l'une quelconque des revendications qui précèdent *caractérisé en ce que* la surface de l'étendue plane de chaque fragment (110) augmente au moins de 300 % lors du mouillage.

9. Article absorbant selon l'une quelconque des revendications qui précèdent *caractérisé en ce que* les fragments (110) sont appliqués contre la surface supérieure de la couche de stockage (106) au moins dans la zone de mouillage.

10. Article absorbant selon l'une quelconque des revendications qui précèdent *caractérisé en ce qu'*au moins l'une des couches de stockage (106) comprend des fibres de cellulose et des particules de superabsorbant, la quantité de matériau superabsorbant calculée par rapport au poids total, à l'état sec, de la couche de stockage (106) étant au moins de 50 % en poids.

11. Article absorbant selon l'une quelconque des revendications 1 à 9 *caractérisé en ce qu'au* moins une des couches de stockage (106) comprend des fibres de cellulose et des particules de superabsorbant, la quantité de matériau superabsorbant calculée par rapport au poids total de la couche de stockage (106), à l'état sec, étant d'au moins 70 % en poids.

FIG.1.

100

104   101

I                                                                          I

102        105  106

108

103

FIG.2.

EP 1 605 881 B1

FIG.3.

13

**EP 1 605 881 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0532002 A
- EP 0615736 A
- EP 0443627 A